# EUROPEAN PATENT APPLICATION

(11) **EP 3 520 791 A1**
(43) Date of publication of application: **07.08.2019**
(21) Application number: 19155516.8
(22) Date of filing: 05.02.2019
(51) Int. Cl.: A61K 31/4422, A61K 31/167, A61K 9/00, A61K 9/06, A61P 1/00, A61P 17/02, A61P 23/02

(54) **NIFEDIPINE-BASED COMPOSITION FOR THE USE IN THE TREATMENT OF ANAL FISSURES AND PROCTALGYA, AND METHOD FOR ADMINISTRATION THEREOF**

(30) Priority: 05.02.2018 IT 201800002413
(71) Applicant: Antropoli, Carmine, 81043 S. Angelo in Formis (IT)
(72) Inventor: Antropoli, Carmine, 81043 S. Angelo in Formis (IT)
(74) Representative: Sarpi, Maurizio

(57) **Abstract**

The invention relates to a topical composition comprising the association of nifedipine and lidocaine, wherein the amount of nifedipine ranges from 0.1% to 0.4% by weight and lidocaine ranges from 1.0% to 2.0% by weight administered by means of single-dose micro-enemas for use in the treatment of anal fissures and proctalgias.

## Description

### Application field of the invention

The present invention relates to the field of pharmaceutical preparations for use in the treatment of functional pathologies of the anal canal, in particular for treating anal fissures and proctalgias.

### Background art

Pathologies and abnormalities of the anal canal, such as hemorrhoids, prolapse of the rectum, fissures, fistulas, abscesses, proctitis (inflammation of the rectum), exudative scars, condylomas, polyps, and tenesmus are highly common disorders. About 30-40% of the population suffers from proctological diseases at least once in life. The disorders deriving therefrom are generally more annoying than dangerous. Therapy can be conservative or surgical. The surgical approach allows to definitively resolve the proctological pathology, while the efficacy of the pharmacological therapy, although preferable because non-invasive, still remains controversial and useful only in the less severe forms, where, in association with a lifestyle change and the application of correct hygienic-behavioral rules, may be sufficient to result in a remission of symptoms and to control the disease.

Drug therapy for treating proctological diseases is based mostly on the administration of antiinflammatory agents as described by W. Klug and H.G. Knoch in Colo-Proctology (1993) 1, pp. 22-28 and by McDonald et al. in the British Journal of Surgery (1983), 70, pp. 25-26.

Specifically, anal fissure is an often unique, solitary linear wound (ulceration) of the anus, and usually located at the back.

There is no specific age of onset. Fissures and cracks are relatively common in newborns. Although the age of greatest incidence is nevertheless juvenile and adult. The incidence is substantially overlapping in the two genders. It is very common in people suffering from constipation and evacuating hard and voluminous stools. However, the fissure can also occur in cases of prolonged diarrhea. In both cases a trauma is generated causing a wound in the anal canal, a wound which can be acute or chronic due to repeated traumas. In the formation of the chronic fissure, the internal sphincter plays an important role, which in all probability causes a localized "micro-ischemia" which slows, and sometimes prevents, the correct healing of the ulceration. The loss of elasticity of the anal canal secondary to previous anal surgery may be a predisposing factor.

Very often the symptomatology is associated with itching even if the pain is the most frequent symptom which appears characteristically at each defecation, often begins at the time of defecation itself and then tends to last from a few minutes until several hours after the evacuation. Here, the presence of abundant nerve endings makes the disorder particularly irritating and painful. The stabbing pain becomes more intense at the time of defecation, especially when hard and voluminous stools are evacuated, which, by friction, favor the lesion of the anal folds. In addition to the aggravation of this annoying disorder, constipation is also one of the main random agents.

Given the diffusion of the constipation problem, it should not be surprising that anal fissures are a condition frequently found in proctological practice.

The presence of fissures causes a spasm of the internal anal sphincter, whose control, unlike to what happens for the outer ring, is independent from the subject's will. This contracture condition is responsible for the chronicization of the disorder, because, in addition to the prevention of the normal and physiological dilatation of the anus, it hinders the blood flow and with it the possibility of spontaneous healing.

Most often, anal fissures are a pathological condition which can be resolved easily and quickly; further, in some patients, the fissures in the perianal area are resolved without the need of pharmacological treatments or special treatments.

When the disorder persists, and tends to become chronic, before resorting to the only possible option represented by surgery (anus plastic surgery, balloon-controlled dilatation or lateral sphincterotomy), the drug treatment is indicated, as well as the correction of eating habits. Diet regularization seems to be a fundamental point to speed up the recovery of the patient: a fiber-rich diet and plenty of water is certainly a valuable aid to facilitate defecation, regularizing the intestinal motility.

Local anesthetics are among the active ingredients most used in therapy against anal fissures, widely used in therapy to reduce the pain associated with anal fissures and hemorrhoids (e.g. lidocaine is often formulated together with steroids to enhance the antiinflammatory effect).

The administration of topical nitroglycerin in the form of an ointment transiently decreases the sphincter pressure at rest by releasing nitric oxide (NO), an endogenous mediator involved in the relaxation of the smooth muscle tone. Therapy can also be continued for 6 months.

The topical application of trinitroglycerin in the anal canal site also acts as a muscle relaxant, reduces anal pressure and increases blood flow, thus reducing pain.

In order to promote healing from anal fissures, injection into the internal and external sphincters of a small botulinum toxin dose directly into the anal sphincter is also practiced, which produces a temporary chemical denervation lasting several months, useful to relax the muscles.

Patent application WO9837886 describes the use of Nifedipine in the treatment of functional pathologies of the anal canal through the administration of topical preparations comprising nifedipine in an amount ranging from 0.5 to 30% by weight, lidocaine hydrochloride in an amount ranging from 0.25 to 15% by weight, carriers and excipients.

Nifedipine is a calcium-antagonist drug belonging to the 1,4-dihydropyridine class. The molecule differs from other calcium-antagonists (e.g. nimodipine) due to its particular activity on the smooth muscle cells of the coronary arteries, as well as those of peripheral resistance vessels. By binding to slow Ca⁺⁺ channels of plasma membrane, nifedipine blocks the transmembrane inflow of the cation within the cell. Due to this action, nifedipine dilates the coronary arteries, reducing the tone of the vascular smooth muscle, preventing vasospasm. The final result is the increase in blood flow in the stenotic segments and the consequent increase in oxygen supply. Nifedipine also reduces the vascular smooth muscle tone of peripheral arterioles, thus reducing peripheral resistance, and hence arterial pressure.

The calcium-antagonist action of Nifedipine together with lidocaine, a substance with local surface anesthetic action, is useful and has given excellent results in combating the symptomatology of the anus fissures by reducing the tone of the internal anal sphincter muscle.

Nifedipine and lidocaine-based treatment of anal fissures should be prolonged for at least 21 days of local administration, applying the cream twice a day.

The nifedipine and lidocaine-based rectal cream Antrolin is currently available on the market and is sold in a 30g aluminum tube with a cannula in a cardboard package.

The methods of administration include that the patient, lying on the bed on his left side:
unscrews the cap from the tube and screws in the special cannula contained in the sales package,
lets out a small cream amount to lubricate the cannula and inserts it in the anus, and
presses the end of the tube again to let out the cream contained in about one centimeter of tube (where one centimeter corresponds to one gram of product).

It is apparent that a treatment method of this type does not facilitate the patient to comply with a precise and constant dosage of the cream over the entire provided duration - three weeks - of the treatment.

Moreover, despite the excellent results of the nifedipine and lidocaine-based therapeutic treatment, similarly to other drugs, it is contraindicated first of all in case of ascertained hypersensitivity to lidocaine (and to any other local anesthetic with the same amide structure) and, obviously, to all the excipients associated with the active ingredients.

In cases of severe nifedipine intoxication, conscience disorders up to coma have been reported, with severe decrease in blood pressure and changes in cardiac rhythms up to cardiogenic shock.

The symptomatology from local anesthetic intoxication (lidocaine first) is borne by the central nervous system: "light head", dizziness, some auditory and visual disorders (up to accommodation difficulties and tinnitus for some slightly more severe case) are the most common symptoms. Depression of the central nervous system and convulsions become part of the most severe symptomatology attributable to this kind of application.

Cases of systemic toxicity in case of overdose in local applications have not been reported. The local application of the nifedipine and lidocaine association, in excessive dosages and/or for too long periods of time, could however induce sensitization and give rise to some local reaction of bleeding and hyperemia.

Although these undesirable effects disappear when treatment is stopped, in order to avoid this disadvantage related to possible overdose and/or inadequate distribution of the nifedipine and lidocaine association at the time of the topical application thereof in the anal region affected by fissures and tenesmus, it would be strongly felt the need to identify a method for administering a Nifedipine and Lidocaine-based rectal cream, which ensures the patient to apply locally the correct doses of the drug for the entire period of the therapeutic treatment, also providing the patient with the relative administration means.

Therefore, it is the object of the present invention to provide a method for administering a composition with a low content of nifedipine through a means, a single-dose micro-enema, with which the therapeutic composition is introduced, being provided with a spout allowing it to be conveyed directly into the area affected by the painful symptom, with single endorectal and perianal applications, in the therapeutically effective amount of said composition, according to the therapy.

The use of single-dose micro-enemas for the specific local treatment of anal fissures by means of Nifedipine and lidocaine-based compositions are not known.

Micro-enemas for topical application of nifedipine and lidocaine-based ointments, circumferentially around the anus, were used only in a double-blind randomized and multicentric trial for the precise purpose of comparing the efficacy of equal amounts of an ointment containing 0.3% nifedipine and 1.5% lidocaine compared to the same amount of an ointment containing only 1.5% lidocaine to alleviate pain in patients who underwent open hemorrhoidectomy, evaluating significant differences in trial groups.

### Summary of the invention

Therefore, the invention first relates to a composition comprising the association of nifedipine and lidocaine wherein the amount of nifedipine ranges from 0.1% to 0.4% by weight and lidocaine ranges from 1.0% to 2.0% by weight, and excipients for its use by micro-enema in the treatment of anal fissures and proctalgias, including rectal tenesmus, anal spasm, hemorrhoids.

Secondly, the invention relates to the administration of the aforementioned composition by means of single-dose micro-enemas, in order to optimize the distribution and delivery of the active ingredient directly where required: a peculiar feature of said micro-enemas is that they are opaque, able to filter light so as not to let ultraviolet rays pass, and to avoid the denaturalization of Nifedipine which, as is known, is photosensitive.

### Detailed description of the invention

According to the present invention, the composition for use in the treatment of anal fissures and proctalgia is characterized by a low concentration of nifedipine compared to the compositions of the state of the art used for the same purpose. This low concentration of one of the two ingredients of the nifedipine and lidocaine association which is the active ingredient for treating anal fissure and anal sphincter tenesmus, is balanced by an improved distribution of the composition in the area where required through a micro-enema, which is the means by which the administration of the composition occurs, favoring the bioavailability of the active ingredient.

By way of example, the formulation of the composition according to the invention is provided below.

### EXAMPLES

### Example 1 - Qualitative and quantitative formulation of the composition

| Ingredient | % g/g |
|---|---|
| White vaseline | 25.50 |
| Propylene glycol | 10.00 |
| Liquid semi-synthetic glycerides | 7.50 |
| Polyethylene glycol stearate 400 | 7.00 |
| Cetostearyl alcohol | 6.00 |
| Glyceryl monostearate | 4.00 |
| Sodium methyl p-oxybenzoate | 0.14 |
| Propyl p-oxybenzoate | 0.06 |
| Nifedipine | 0.30 |
| Lidocaine hydrochloride | 1.50 |
| Purified water q.s. to | 100 |

The composition according to the invention is in the form of a solution with different degree of viscosity, preferably in the form of a cream, gel, ointment, which is contained, according to a peculiar feature of the invention, rather than in tubes or jars from which to extract, manually, the therapeutically effective amount, in dark walls polyethylene single-dose micro-enemas, hermetically sealed, which allow the composition to maintain its chemical-physical stability over time, since nifedipine is highly photosensitive.

### Example 2 - Clinical trial

This trial was conducted according to a prospective, randomized, double-blind, multicentric plan, in order to compare the efficacy of two different methods for locally applying a nifedipine and lidocaine gel-based composition in resulting in acute anal fissure healing by relaxing the internal anal sphincter.

One hundred and twenty patients were recruited in the trial, of which 48 females and 72 males with anal fissures and proctalgias of various nature who gave informed consent to the trial after being informed of the practice they were about to undergo; they have received a clinical examination. A questionnaire was administered to evaluate the symptoms and the pain, and a proctoscopy and an anorectal manometry were performed.

Patients were divided into two groups, a first group (n = 60 of which 23 females and 37 males) was treated with the manual local application of a composition having the formulation of example 1 comprising a 0.3% nifedipine and 1.5% lidocaine gel every 12 hours for three weeks; the suggested manual application was about 1 cm³ of cream, corresponding to 2.5-3 g of cream amount. The second group comprising 60 patients (of which 25 females and 35 males) received the administration by means of an opaque micro-enema of 3 g of a 0.3% nifedipine and 1.5% lidocaine gel every 12 hours for three weeks. Manometry was performed before and on days 14 and 21. Anal pressures were measured by recording the resting and compression pressures.

### Results

The obtained results showed total remission from acute anal fissure reached after 15 days of therapy in 95% of patients treated with nifedipine and lidocaine administered with micro-enemas (P <0.01), compared to the first group wherein 90% of patients obtained remission in 21 days (P <0.01). No difference was obtained in the decrease of previously elevated maximum resting anal pressures passing from a mean value ± standard deviation of 72.5 ± 10.07 mmHg to 50.5 ± 10.03 mmHg in both groups when total remission was reached. This represents an average reduction of 30 percent (P <0.01). A significant decrease in compression pressures was also observed in both groups (from a mean value ± standard deviation of 130.5 ± 19.25 mmHg to 108.5 ± 18.55 mmHg, an average reduction of 16.8%, P <0.01). No systemic side effects or significant anorectal bleeding were observed in treated patients.

From these results it follows that:
- patients in group B had a total or almost total remission of symptoms in 95% with an increase in No responders or partial remission of the underlying disease of 5% compared to 10% of group A.
- the timing of remission of group B disease was found to be lower than group A of about 7 days.
- the practicality of the micro-enema with a constant content of 3 g of cream of the B group was appreciated by the subjects who brought their satisfaction rating to over 96%.

It is therefore apparent that the micro-enema formulation is not only preferable for the correct dosage required for the remission of the underlying pathology but also, given the comfort of single-dose micro-enemas, is a more hygienic solution for the patient, with greater ease of use and with an always constant administration of the active ingredients. This statement highlights the importance of treatment B also in terms of overall health savings: in fact, patients in group B were found to be less absentee in the workplace due to the practicality of the micro-enema.

In addition, it has been seen at the weekly controls how the remission from the disease was reached in less time in the subjects of group B than those of group A. Such variation of timing in the remission of the disease could find explanation in the fact that the single tube of treatment A does not allow a constant administration of the drug leading the patient to an irregular administration of the product. This obviously results in a sub-dosing of the active ingredients during each application.

### CONCLUSIONS

Our trial clearly demonstrates that the administration of a low-dose nifedipine (0.3%) composition in association with lidocaine by means of a micro-enema dispensing about 3 g of composition provides a more rapid and lasting therapeutic effect than the manual application of the same composition with important and favorable consequences both on the quality of life of patients since it allows to use the right amounts of active ingredient every time and ensures a constant dosage during each application, and for the resulting health saving.

## Claims

1. A rectal composition comprising the association of nifedipine and lidocaine, wherein the amount of nifedipine ranges from 0.1% to 0.4% by weight and lidocaine ranges from 1.0% to 2.0% by weight for use in the treatment by means of single-dose micro-enemas of anal fissures and proctalgias, rectal tenesmus, anal spasm, hemorrhoids.

2. A composition according to claim 1 having the following formulation:
| | |
|---|---|
| White Vaseline | 25.50 % w/w |
| Propylene glycol | 10.00 |
| Liquid semi-synthetic glycerides | 7.50 |
| Polyethylene glycol stearate 400 | 7.00 |
| Cetostearyl alcohol | 6.00 |
| Glyceryl monostearate | 4.00 |
| Sodium methyl p-oxybenzoate | 0.14 |
| Propyl p-oxybenzoate | 0.06 |
| Nifedipine | 0.30 |
| Lidocaine hydrochloride | 1.50 |
| Purified water | q.s. to 100. |

3. A rectal composition for treating anal fissures and proctalgias according to any one of the preceding claims which is in the form of a cream, gel, ointment, liniment.

4. A method for administering the composition according to any one of claims 1 to 3 **characterized in that** it includes:
providing said composition in single-dose micro-enemas;
using said micro-enemas for a single application in order to optimize the distribution and delivery of the active ingredient directly where required;
said micro-enemas being opaque or anyway able to avoid light to pass, so as not to denature nifedipine contained in the composition.
